# DEMANDE DE BREVET EUROPEEN

(11) **EP 0 778 007 A1**
(43) Date de publication de la demande: **11.06.1997**
(21) Numéro de dépôt: 96420350.9
(22) Date de dépôt: 06.12.1996
(51) Int. Cl.: A61B 17/70

(54) **Dispositif d'assemblage pour pièces allongées de matériel d'ostéosynthèse, notamment rachidienne**

(30) Priorité: 07.12.1995 FR 9514735
(71) Demandeur: Groupe Lepine, F-69003 Lyon (FR)
(72) Inventeur: Pfaifer, Patrick, 69003 Lyon (FR)
(74) Mandataire: Maureau, Philippe

(57) **Abrégé**

Ce dispositif comprend un corps (10), une bague (11) et un écrou (12) ;
- le corps présente :
   une portion supérieure filetée (10a),
   deux pattes (15) délimitant entre elles un canal (16) de réception de l'une (4) des pièces allongées (4,5) à assembler, ces pattes (15) étant mobiles en écartement ou en rapprochement l'une par rapport à l'autre ;
   au moins une surface d'appui (19) aménagée sur la face latérale du corps (10) ou des pattes (15), formant une butée axiale, et
   une lumière transversale (20) dans laquelle peut être engagée la deuxième pièce allongée (5) à assembler ;
- la bague (11) est apte à être engagée sur le corps (10) jusqu'à venir prendre appui sur la butée (19), et est dimensionnée de manière à venir, dans cette position, ceinturer les pattes (15)
- l'écrou (12) est apte à être vissé sur la portion supérieure filetée (10a) du corps (10).

## Description

La présente invention concerne un dispositif d'assemblage pour pièces allongées de matériel d'ostéosynthèse, notamment rachidienne. Deux dispositifs selon l'invention sont en particulier destinés à réaliser l'assemblage de deux tiges d'étayage du rachis et d'une traverse reliant ces tiges transversalement.

Un matériel d'ostéosynthèse rachidienne comprend généralement deux tiges d'étayage, disposées parallèlement l'une à l'autre de part et d'autre des vertèbres, des organes d'ancrage de ces tiges aux vertèbres, tels des crochets ou des vis pédiculaires, et des traverses qui relient transversalement ces tiges de proche en proche pour les maintenir l'une par rapport à l'autre.

Il est connu de réaliser l'assemblage d'une traverse avec deux tiges d'étayage au moyen d'embouts en forme de crochet vissés sur les extrémités de la traverse, dont les parties recourbées sont engagées autour des tiges.

Ces dispositifs ont pour inconvénients d'être relativement longs et difficiles à mettre en place et de ne permettre qu'un assemblage relativement peu rigide en flexion et en torsion.

D'autres dispositifs prévus pour réaliser un tel assemblage comprennent une pluralité de pièces de petites dimensions, notamment des vis ou écrous de serrage. La mise en place et le serrage de ces différentes pièces sont longs et guère faciles à réaliser.

Certains dispositifs présentent des surfaces de contact avec les tiges et/ou la traverse relativement limitées, ce qui peut induire à la longue des risques de glissement ou de prise de jeu sous l'effet des contraintes répétées que le matériel subit lors des mouvements du patient.

L'invention vise à remédier à l'ensemble de ces inconvénients, en fournissant un dispositif facile et rapide à mettre en place, présentant une bonne rigidité en torsion et en flexion et éliminant tout risque de glissement ou de prise de jeu entre les pièces assemblées.

Le dispositif selon l'invention comprend un corps, une bague et un écrou ;
- le corps présente :
   une portion supérieure filetée,
   deux pattes faisant saillie de son extrémité inférieure, délimitant entre elles un canal de réception de l'une des pièces allongées à assembler, ces pattes étant mobiles en écartement ou en rapprochement l'une par rapport à l'autre, de manière à autoriser, dans une position d'écartement relatif, l'introduction dans ledit canal de la pièce allongée à assembler, et à empêcher, dans une position de rapprochement relatif, le retrait de cette pièce allongée hors dudit canal ;
   au moins une surface d'appui aménagée sur la face latérale du corps ou des pattes, formant une butée axiale, et
   une lumière transversale dans laquelle peut être engagée la deuxième pièce allongée à assembler ;
- la bague est apte à être engagée sur le corps jusqu'à venir prendre appui sur la butée, et est dimensionnée de manière à venir, dans cette position, ceinturer les pattes et les maintenir dans la position précitée de rapprochement relatif ;
- l'écrou est apte à être vissé sur la portion supérieure filetée du corps,

l'ensemble étant conformé de manière à ce que l'écrou, au cours de son vissage, vienne serrer ladite deuxième pièce allongée à assembler et la bague contre la butée précitée.

Le serrage de l'écrou permet ainsi de maintenir la bague autour des pattes, donc de maintenir les pattes dans leur position de rapprochement relatif permettant la rétention de la première pièce allongée à assembler, et permet simultanément de serrer la deuxième pièce à assembler entre lui-même et la bague, et donc d'assurer l'immobilisation de cette deuxième pièce et son assemblage avec la première pièce.

Ce dispositif a pour avantage d'être facile et rapide à mettre en place, en particulier sur des tiges d'étayage après que celles-ci aient été fixées au rachis.

Les trois pièces constituant ce dispositif ont des dimensions permettant des manipulations faciles. En outre, les pattes et la lumière précitées ont des surfaces de contact importantes avec les pièces allongées à assembler, ce qui élimine tout risque de glissement ou de prise de jeu entre ces pièces.

Avantageusement, la butée et la bague sont conformées pour réaliser un rapprochement des pattes l'une vers l'autre lors du serrage de l'écrou, de manière à réaliser un serrage des pattes sur la pièce allongée à assembler. Ce serrage permet d'obtenir une parfaite immobilisation de cette pièce par rapport aux pattes, propre à résister aux contraintes exercées sur le matériel lors des mouvements du patient.

Selon une forme de réalisation préférée de l'invention dans ce cas, la butée est constituée par un épaulement conique et la bague comprend un épaulement conique complémentaire. Le déplacement de ces épaulements l'un par rapport à l'autre lors du serrage de l'écrou permet de déplacer les pattes l'une en direction de l'autre.

De préférence, la bague est conformée de manière à venir en appui sur ladite première pièce à assembler lors du serrage de l'écrou. Elle permet ainsi d'augmenter les surfaces de contact du dispositif avec cette pièce.

Selon une forme de réalisation préférée de l'invention dans ce cas, la bague comprend deux encoches aménagées dans son bord inférieur, diamétralement opposées l'une à l'autre.

Avantageusement, la bague comprend deux encoches aménagées dans son bord supérieur, destinées à venir en regard de la lumière du corps recevant ladite deuxième pièce allongée à assembler. Ces encoches favorisent le guidage de cette deuxième pièce à assembler, pour son engagement dans la lumière.

Ces différentes encoches permettent de réduire la hauteur du dispositif d'assemblage. Les encoches aménagées dans le bord inférieur de la bague permettent de positionner instantanément les deux autres encoches en face de la lumière du corps. En outre et surtout, ces encoches permettent de caler la bague par rapport aux deux pièces allongées à assembler, de sorte que la bague constitue un moyen supplémentaire de maintien de ces pièces et de répartition des efforts de flexion ou de torsion que le dispositif peut être amené à subir.

Avantageusement, les pattes comprennent, sur leurs faces en regard et au niveau de leurs extrémités libres, des bourrelets en saillie situés à une distance l'un de l'autre inférieure à la largeur de la pièce allongée à assembler destinée à être reçue dans le canal.

Ces bourrelets permettent un encliquetage de cette pièce entre les pattes, de sorte que le corps du dispositif peut être maintenu provisoirement sur la pièce à assembler le temps que la bague et l'écrou soient mis en place.

De préférence, le corps comprend une fente longitudinale médiane débouchant dans le fond du canal délimité par les pattes, cette fente étant propre à augmenter les possibilités de déplacement des pattes l'une par rapport à l'autre, notamment pour faciliter leur serrage ou leur encliquetage.

Pour sa bonne compréhension, l'invention est à nouveau décrite ci-dessous en référence au dessin schématique annexé représentant, à titre d'exemple non limitatif, une forme de réalisation préférée du dispositif qu'elle concerne.
La figure 1 est une vue en perspective de deux dispositifs assurant l'assemblage de pièces allongées que comprend un matériel d'ostéosynthèse rachidienne ;
la figure 2 est une vue en perspective des différents éléments composant ce dispositif, et de deux pièces devant être assemblée par ce dispositif ;
la figure 3 est une vue en perspective de ce dispositif et de ces pièces, après assemblage, et
la figure 4 est une vue de ce dispositif, en coupe selon la ligne IV-IV de la figure 3.

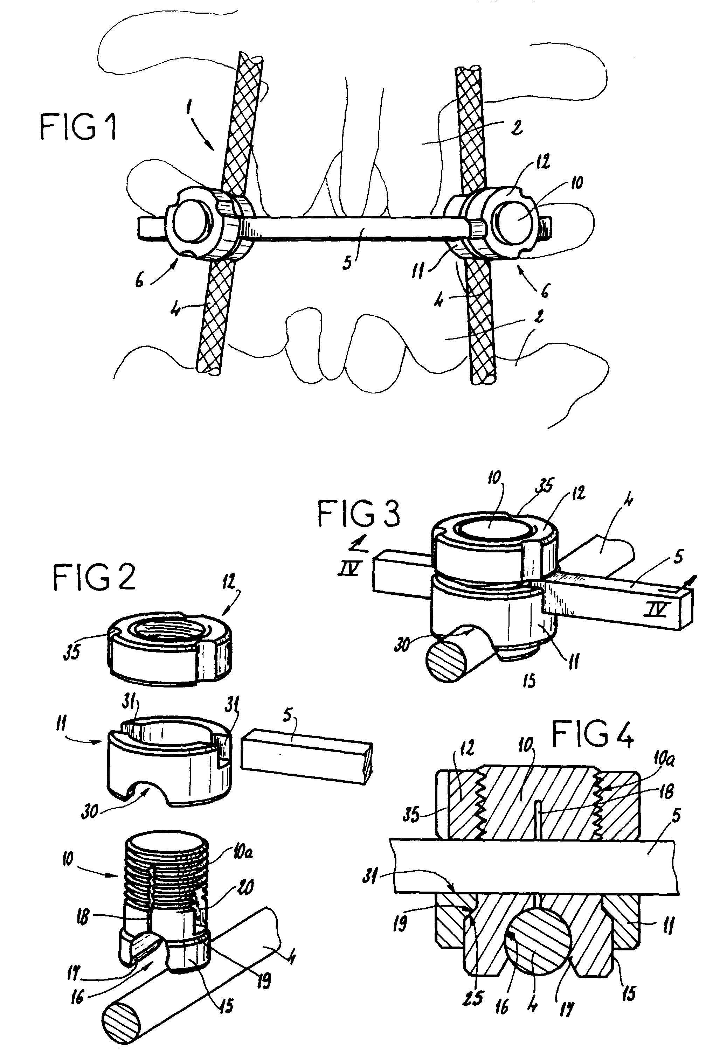

La figure 1 représente une portion d'un matériel d'ostéosynthèse rachidienne 1, implanté sur les vertèbres 2 d'un rachis.

Le matériel 1 comprend deux tiges d'étayage 4, disposées parallèlement l'une à l'autre de part et d'autre des vertèbres 2, des organes d'ancrage de ces tiges 4 aux vertèbres 2, tels des crochets ou des vis pédiculaires (non représentés car bien connus en eux-mêmes), au moins une traverse 5 qui relie transversalement ces tiges 4 pour les maintenir l'une par rapport à l'autre, et au moins deux dispositifs 6 réalisant l'assemblage des tiges 4 et de la traverse 5.

Ainsi que cela apparaît sur les figures 2 à 4, le dispositif 6 comprend un corps 10, de forme générale cylindrique, une bague 11 et un écrou 12.

Le corps 10 présente :
- une portion supérieure filetée 10a ;
- deux pattes 15 faisant saillie de son extrémité inférieure, délimitant entre elles un canal circulaire 16 de réception d'une tige 4 ; ces pattes 15 comprennent, sur leurs faces en regard et au niveau de leurs extrémités libres, des bourrelets 17 en saillie situés à une distance l'un de l'autre inférieure au diamètre des tiges 4 ;
- une fente longitudinale médiane 18 débouchant dans le fond du canal 16 ;
- un épaulement conique 19 aménagé sur la face latérale extérieure des pattes 15, formant une butée axiale, et
- une lumière transversale 20 au travers de laquelle peut être engagée la traverse 5 ; cette lumière 20 présente une hauteur supérieure à celle de la traverse 5 et s'étend en partie le long de la portion filetée 10a.

La bague 11 présente un diamètre interne correspondant, au jeu près, au diamètre du corps 10, et comprend un épaulement conique 25 à sa partie inférieure apte à venir en butée contre l'épaulement 19 lorsque la bague 11 est engagée sur le corps 10.

La bague 11 se prolonge vers le bas au-delà de l'épaulement 25, ainsi que le montre la figure 4, de sorte qu'elle vient ceinturer les pattes 15 lorsque l'épaulement 25 vient en appui contre l'épaulement 19.

En outre, la bague 11 comprend deux encoches hémi-circulaires 30 aménagées dans son bord inférieur, diamétralement opposées l'une à l'autre, et deux encoches 31 de forme carrée aménagées dans son bord supérieur, également disposées de manière diamétralement opposée l'une par rapport à l'autre. Les encoches 31 sont disposées perpendiculairement aux encoches 30.

L'écrou 12 est apte à être vissé sur la portion filetée 10a. Pour sa manoeuvre en rotation, il comprend trois cannelures 35 aménagées dans sa paroi périphérique, destinées à recevoir les ergots correspondants d'une clef à tube (non représentée).

En pratique, les vis ou crochets pédiculaires sont mis en place dans les vertèbres 2 et les tiges 4 sont fixées sur eux.

Pour l'assemblage d'une traverse 5 aux tiges 4, les corps 10 de deux dispositifs 6 sont encliquetés sur chacune des tiges 4, en face l'un de l'autre. Cet encliquetage est rendu possible grâce à la fente 18, qui permet une certaine mobilité des pattes 15 en écartement l'une par rapport à l'autre, grâce aux bourrelets 17, qui permettent l'écartement des pattes 15 lors de l'introduction des tiges 4 dans les canaux 16 et la rétention de ces tiges 4 dans ces canaux 16 une fois cette introduction réalisée, et grâce au rappel élastique des pattes 15 dans leur position d'origine, résultant de la nature du matériau constituant le corps 10.

Chaque bague 11 est ensuite introduite sur le corps 10 correspondant, jusqu'à ce que son épaulement 25 vienne en appui contre l'épaulement 19. Dans cette position d'appui, les encoches 30 sont engagées autour de la tige 4, et les encoches 31 sont placées en regard des ouvertures latérales de la lumière 20.

La traverse 5 est ensuite engagée au travers de la lumière 20. Les encoches 31 facilitent cette introduction.

Enfin, l'écrou 12 est vissé sur la portion filetée 10a.

Cet écrou 12, au cours de son vissage, vient serrer la traverse 5 et la bague 11 contre l'épaulement 19.

Ce serrage provoque un léger glissement de l'épaulement 25 par rapport à l'épaulement 19, ce qui provoque le rapprochement des pattes 15 l'une vers l'autre, permettant de réaliser un serrage de ces pattes 15 sur la tige 4. Simultanément, l'écrou 12 vient serrer la traverse 5 entre lui-même et la bague 11, et permet d'assurer l'immobilisation de cette traverse 5 et donc son assemblage avec la tige 6.

L'invention fournit un dispositif perfectionné d'assemblage de pièces allongées de matériel d'ostéosynthèse, notamment rachidienne, ayant les nombreux avantages indiqués plus haut, notamment ceux d'être facile et rapide à mettre en place, de présenter une bonne rigidité en torsion et en flexion et d'éliminer tout risque de glissement ou de prise de jeu entre les pièces assemblées.

## Revendications

1. Dispositif d'assemblage pour pièces allongées (4,5) de matériel d'ostéosynthèse, notamment rachidienne, caractérisé en ce qu'il comprend un corps (10), une bague (11) et un écrou (12) ;
- le corps (10) présente :
une portion supérieure filetée (10a),
deux pattes (15) faisant saillie de son extrémité inférieure, délimitant entre elles un canal (16) de réception de l'une (4) des pièces allongées à assembler, ces pattes (15) étant mobiles en écartement ou en rapprochement l'une par rapport à l'autre, de manière à autoriser, dans une position d'écartement relatif, l'introduction dans ledit canal (16) de la pièce allongée (4) à assembler, et à empêcher, dans une position de rapprochement relatif, le retrait de cette pièce allongée (4) hors dudit canal (16) ;
au moins une surface d'appui (19) aménagée sur la face latérale du corps (10) ou des pattes (15), formant une butée axiale, et
une lumière transversale (20) dans laquelle peut être engagée la deuxième pièce allongée (5) à assembler ;
- la bague (11) est apte à être engagée sur le corps (10) jusqu'à venir prendre appui sur la butée (19), et est dimensionnée de manière à venir, dans cette position, ceinturer les pattes (15) et les maintenir dans la position précitée de rapprochement relatif ;
- l'écrou (12) est apte à être vissé sur la portion supérieure filetée (10a) du corps (10),
l'ensemble étant conformé de manière à ce que l'écrou (12), au cours de son vissage, vienne serrer ladite deuxième pièce allongée (5) à assembler et la bague (11) contre la butée (19) précitée.

2. Dispositif selon la revendication 1, caractérisé en ce que la butée (19) et la bague (11) sont conformées pour réaliser un rapprochement des pattes (15) l'une vers l'autre lors du serrage de l'écrou (12), de manière à réaliser un serrage des pattes (15) sur la pièce allongée (4) à assembler.

3. Dispositif selon la revendication 2, caractérisé en ce que la butée est constituée par un épaulement conique (19) et en ce que la bague (11) comprend un épaulement conique (25) complémentaire.

4. Dispositif selon l'une des revendications 1 à 3, caractérisé en ce que la bague (11) est conformée de manière à venir en appui sur ladite première pièce (4) à assembler lors du serrage de l'écrou (12).

5. Dispositif selon la revendication 4, caractérisé en ce que la bague (11) comprend deux encoches (30) aménagées dans son bord inférieur, diamétralement opposées l'une à l'autre.

6. Dispositif selon l'une des revendications 1 à 5, caractérisé en ce que la bague (11) comprend deux encoches (31) aménagées dans son bord supérieur, destinées à venir en regard de la lumière (20) du corps (10) recevant ladite deuxième pièce allongée (5) à assembler.

7. Dispositif selon l'une des revendications 1 à 6, caractérisé en ce que les pattes (15) comprennent, sur leurs faces en regard et au niveau de leurs extrémités libres, des bourrelets (17) en saillie situés à une distance l'un de l'autre inférieure à la largeur de la pièce allongée (4) à assembler destinée à être reçue dans le canal (16).

8. Dispositif selon l'une des revendications 1 à 7, caractérisé en ce que le corps (10) comprend une fente longitudinale médiane (18) débouchant dans le fond du canal (16) délimité par les pattes (15), cette fente (18) étant propre à augmenter les possibilités de déplacement des pattes (15) l'une par rapport à l'autre.
